# EUROPEAN PATENT SPECIFICATION

(11) **EP 2 886 053 B1**
(45) Date of publication and mention of the grant of the patent: **22.09.2021**
(21) Application number: 13876768.6
(22) Date of filing: 09.08.2013
(51) Int. Cl.: G01R 33/563, A61B 5/055

(54) **MAGNETIC RESONANCE IMAGING DEVICE FOR DIFFERENTIATION OF MYOCARDIAL TISSUES**
MAGNETRESONANZBILDGEBUNGSVORRICHTUNG ZUR UNTERSCHEIDUNG VON MYOCARDGEWEBEN
DISPOSITIF D'IMAGERIE PAR RÉSONANCE MAGNÉTIQUE POUR LA DIFFÉRENCIATION DES TISSUS MYOCARDIQUES

(30) Priority: 16.08.2012 US 201213587294
(43) Date of publication of application: 24.06.2015
(73) Proprietor: Toshiba Medical Systems Corporation, Otawara-shi, Tochigi-ken 324-0036 (JP)
(72) Inventor: MIYAZAKI, Mitsue, Vernon Hills, Illinois 60061 (US); ZHOU, Xiangzhi, Vernon Hills, Illinois 60061 (US); HOSHINO, Tsutomu, Vernon Hills, Illinois 60061 (US)
(74) Representative: Moreland, David
(86) International application number: PCT/JP2013/071733
(87) International publication number: WO 2014/027629

(56) References cited:
- EP-A1- 2 299 286
- JP-A- 2000 342 555
- JP-A- 2001 231 764
- JP-A- 2010 022 813
- JP-A- 2010 201 154
- JP-A- 2011 067 696
- JP-A- 2011 083 592
- US-A1- 2010 198 053
- CALAMANTE F ET AL: "Measuring cerebral blood flow using magnetic resonance imaging techniques", JOURNAL OF CEREBRAL BLOOD FLOW & METABO, NATURE PUBLISHING GROUP, US, vol. 19, no. 7, 1 July 1999 (1999-07-01), pages 701-735, XP009160891, ISSN: 0271-678X, DOI: 10.1097/00004647-199907000-00001
- None

## Description

### Field

Embodiments relate to a magnetic resonance imaging apparatus.

### Background

Conventionally, a method called myocardial perfusion is known as a cardiac imaging method using magnetic resonance imaging. Myocardial perfusion is a method of successively imaging T1-contrast images at high speed and thus observing, as a change in time, impregnation of a gadolinium (Gd)-based contrast agent in myocardium in order to evaluate the blood supply to the myocardium. EP2299286 discloses an MRI apparatus including an imaging data-acquiring unit and a blood flow information generating unit. The imaging data-acquiring unit acquires imaging data from an imaging region including myocardium, without using a contrast medium, by applying a spatial selective excitation pulse to a region including at least a part of an ascending aorta for distinguishably displaying inflowing blood flowing into the imaging region. The blood flow information generating unit generates blood flow image data based on the imaging data.
US2010198053A1 discloses a magnetic resonance imaging apparatus includes a first blood flow image acquisition unit and a second blood flow image acquisition unit. The first blood flow image acquisition unit acquires a first blood flow image of a breast of an object without contrast medium. The second blood flow image acquisition unit acquires a second blood flow image without contrast medium with applying a spin labeling pulse by which a region to be tagged is set based on the first blood flow image as a reference image so as to distinguish blood flowing into a desired region.

### Brief Description of Drawings

FIG. 1 is a high-level schematic block diagram of an MRI system of an exemplary embodiment configured to provide non-contrast cardiac MRI with a function of differentiating between ischemic, infarct and normal myocardial tissues.
FIG. 2A is a schematic depiction of a first example of a tag-on, tag-off MRI data acquisition sequence for use in the MRI system of the exemplary embodiment.
FIG. 2B is a schematic depiction of a second example of a tag-on, tag-off MRI data acquisition sequence for use in the system of the exemplary embodiment.
FIG. 3 is a diagram of a region to which a tag pulse is applied in the exemplary embodiment.
FIG. 4A is a schematic illustration of acquisition of tag-on/tag-off 2D/3D k-space data, 2D/3D Fourier transformation of the acquired k-space data, and subtraction of the reconstructed tag-on and tag-off image data so as to produce blood perfusion image data in at least one region of interest.
FIG. 4B is a schematic depiction of a tag-on and tag-off MRI data acquisition sequence that is executed for multiple TI times.
FIG. 5 is a schematic depiction of myocardial tissue segmentation for a cross-sectional image of a left ventricular myocardial short axis.
FIG. 6 is an exemplary plot where acquired MR signal strength (representing blood perfusion) is plotted as a function of time, illustrating how non-contrast cardiac MRI perfusion data for a given region of interest (perhaps a region of interest down to a single pixel ROI) can be analyzed to distinguish between normal, ischemic and infarct myocardial tissues - as well as a revascularized infarct (treated) myocardial tissue.
FIG. 7 is a flowchart illustrating a computer program code structure for implementing exemplary embodiments of non-contrast myocardial MRI capable of distinguishing between ischemic, infarct and normal myocardial tissues based upon peak flow time and/or peak flow amplitude and/or integrated flow quantity.
FIG. 8 is a schematic depiction of a bSSFP tag-on MRI data acquisition sequence.
FIG. 9 is a schematic depiction of a bSSFP tag-off MRI data acquisition sequence.
FIG. 10 is a schematic depiction of a tag-on/tag-off alternating MRI data acquisition sequence.

### Description of Embodiments

A magnetic resonance imaging apparatus according to an embodiment comprises a sequence controller and an analyzer. The sequence controller is configured to perform data acquisition in an imaging region after multiple given times since an application of a tag pulse to a tag region, with the multiple given times being different to each other. The analyzer is configured to use multiple sets of the acquired data corresponding to the multiple given times to analyze changes in signal strength over time since the application of the tag pulse. A magnetic resonance imaging apparatus according to the embodiments (hereinafter, "MRI (magnetic resonance imaging) system" as appropriate) will be described with reference to the drawings. Embodiments are not limited to the following embodiments. The contents of descriptions of an embodiment are applicable to other embodiments as well in principle.

The MRI system according to the embodiment provides a non-contrast MRI with a function of differentiating between ischemic, infarct and normal tissues of myocardium.

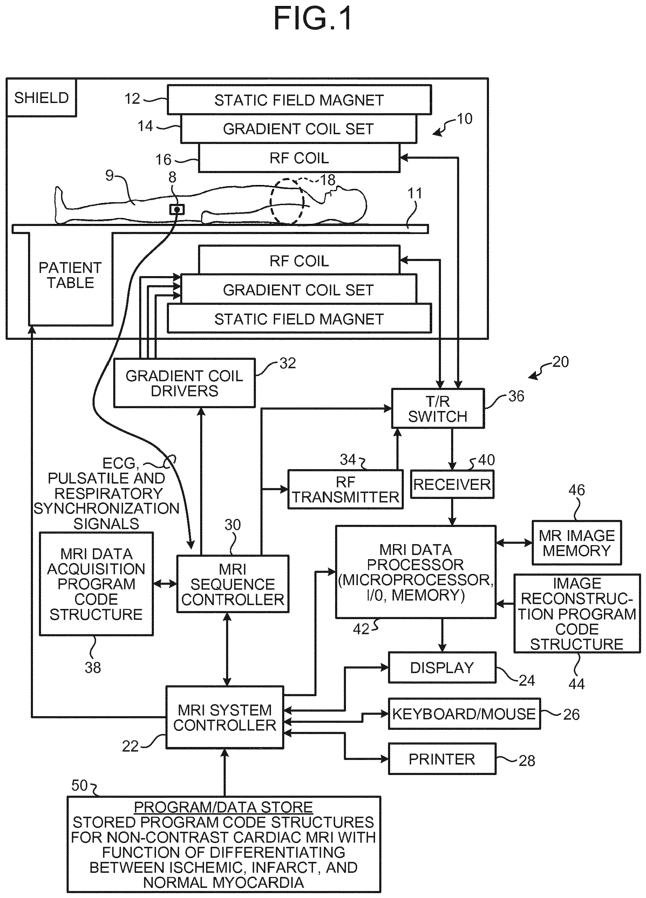

FIG. 1 is a block diagram showing a configuration of the MRI system according to the embodiment. As shown in FIG. 1, the MRI system includes a gantry 10 (shown in schematic cross-section) and various related system components 20 interfaced with the gantry 10. At least the gantry 10 is typically located in a shielded room. The MRI system geometry depicted in FIG. 1 includes a substantially coaxial cylindrical structure of a static field Bo magnet 12, a Gx, Gy and Gz gradient coil set 14 and a RF (radio frequency) coil assembly 16. Along the horizontal axis of this cylindrical array of elements is an imaging region 18 shown as substantially encompassing the anatomic tissue of interest (i.e., region of interest "ROI") for a patient 9 (e.g., the thorax for cardiac MRI) supported by a patient couch or table 11.

An MRI system controller 22 has input/output ports connected to a display 24, a keyboard/mouse 26 and a printer 28. As will be appreciated, the display 24 may be of the touch-screen variety so that it provides a function of inputting control signals as well.

The MRI system controller 22 interfaces with an MRI sequence controller 30. The MRI sequence controller 30 controls a Gx, Gy and Gz gradient coil drivers 32, as well as a RF transmitter 34 and transmit/receive switch 36 (if the same RF coil is used for both transmission and reception). As those skilled in the art will appreciate, many different types of RF coils (e.g., whole body coils, surface coils, birdcage coils, coil arrays, etc.) may be employed to transmit and/or receive RF signals to/from the ROI in the imaging volume. As will also be appreciated, one or more suitable physiological transducers 8 may be affixed to the patient's body to provide ECG (electrocardiogram), respiratory and/or pulsatile synchronization signals to the MRI sequence controller 30. The MRI sequence controller 30 also has access to a suitable program code structure 38 for implementing MRI data acquisition sequences already available in the repertoire of the MRI sequence controller 30. For example, operator and/or system inputs defining particular MRI data acquisition sequence parameters, one or more ROI, etc. are used to generate non-contrast cardiac MRI tissue images.

The MRI system 20 includes an RF receiver 40 providing input to a data processor 42 so as to generate processed image data which may be sent to the display 24. The MRI data processor 42 is also configured for access to an image reconstruction program code structure 44 and to a MR image memory 46 (e.g., for storing MR image data derived from processing in accordance with the exemplary embodiments and the image reconstruction program code structure 44).

Also illustrated in FIG. 1 is a generalized depiction of an MRI system program/data store 50 where stored program code structures (e.g., for non-contrast cardiac MRI capable of differentiating between ischemic, infarct and normal myocardial tissues), as well as a related graphical user interface (GUI), operator inputs to same, etc., are stored in computer readable storage media accessible to the various data processing components of the MRI system. As those in the art will appreciate, the program storage 50 may be segmented and directly connected, at least in part, to different processing computers among the processing computers of the MRI system 20, having most immediate need for such stored program code structures in their normal operation (i.e., rather than being commonly stored and connected directly to the MRI system controller 22) .

Indeed, as those skilled in the art will appreciate, FIG. 1 is a very high-level diagram of a typical MRI system displayed as simplified with some modifications so as to practice embodiments to be described hereinbelow. The system components can be divided into different logical processing collections of "boxes" and typically comprise numerous digital signal processors (DSP), microprocessors, special purpose processing circuits (e.g., for fast A/D conversions, fast Fourier transforming, array processing, etc.). Each of those processors is typically a clocked "state machine" wherein the physical data processing circuits progress from one physical state to another upon the occurrence of each clock cycle (or predetermined number of clock cycles).

Not only does the physical state of processing circuits (e.g., CPUs (central processing units), registers, buffers, arithmetic units, etc.) progressively change from one clock cycle to another during the course of operation, the physical state of associated data storage media (e.g., bit storage sites in magnetic storage media) is transformed from one state to another during operation of such a system. For example, at the conclusion of an MR imaging reconstruction process, an array of computer-readable accessible data value storage sites (e.g., multi-digit binary representations of pixel values) in physical storage media will be transformed from some prior state (e.g., all uniform "zero" values or all "one" values) to a new state. In this case, the physical states at the physical sites of such an array (e.g., of pixel values) vary between minimum and maximum values to represent real world physical events and conditions (e.g., the tissues of a patient over an imaged region space). As those in the art will appreciate, such arrays of stored data values represent and also constitute a physical structure - as does a particular structure of computer control program codes that, when sequentially loaded into instruction registers and executed by one or more CPUs of the MRI system 20, cause a particular sequence of operational states to occur and be transitioned through within the MRI system.

The exemplary embodiments described below provide improved ways to acquire MRI data, and/or to process MRI data acquisitions, and/or to generate and display MR images.

In the above-described MRI system, for example, the MRI sequence controller 30 performs data acquisition in an imaging region after multiple given times (e.g., TI (Time to Inversion) time) since an application of a tag pulse to a tag region, with the multiple given times being different to each other. Furthermore, for example, the MRI system controller 22 (also referred to as "analyzer") use multiple sets of the acquired data corresponding to the multiple given times to analyze changes in signal strength over time since the application of the tag pulse, which will be described in detail below.

Use of an injected gadolinium (Gd)-based contrast agent (possibly in association with an injected stress perfusion agent) is known, but use of such contrast agents is often not an acceptable MRI technique for detecting infarct and ischemic lesions in myocardium. Late gadolinium contrast enhancement (LGE) is based on measuring a difference between normal and infarct myocardium by observing T1-contrast of the gadolinium contrast agent via observed MR signals from the myocardium after injection. In order to get a realistic observation result of myocardium under stress, such stress may be induced by patient physical exercise or injected drug-induced stress (intended to cause increase in heart rate, dilation of cardiovascular blood vessels, etc., similar to that caused by physical exercise) so as to hopefully better observe blood flow abnormalities under such transient stress situations.

We have now discovered a way to avoid use of such contrast agents by achieving a non-contrast (i.e., without injection of a chemical contrast agent) MRI technique for producing myocardium perfusion curves/table data that can be used to distinguish between normal myocardium, ischemic myocardium and infarct myocardium. Indeed, even revascularized infarct myocardium (treated with revascularization techniques such as stent insertions into blood vessels, surgical bypass blood vessel operations, etc.) may be distinguished. In accordance with representative embodiments, such myocardial state distinctions can be made within any desired region of interest (e.g., an operator-defined arbitrary region of interest, a standard AHA (American Heart Association) segment, a single pixel, etc.).

Although injection of a contrast agent (e.g., gadolinium-based) is avoided, it may still be desirable to carry out representative embodiments in combination with patient stress (either exercise-induced or drug-induced) so as to better detect abnormalities that may exist or be prominent only during such stressed situations.

In exemplary embodiments, a set of "tag-on" k-space data (2D or 3D) is acquired after an incoming volume of blood has been "tagged" with an spatially selective RF pulse (e.g. typically a spatially selective 180° inversion pulse). The data acquisition subsequence starts after a given time to inversion (TI). Similar sets of "tag-off" MRI data are also acquired using the same TI time- but without the initial spatially selective RF tagging pulse. This technique is sometimes known in the art as BBTI (black blood time to inversion) imaging. A sequence of such tag-on/tag-off data sets for each of multiple TI times is acquired in k-space.

For each given TI time, subtraction is performed (e.g., on a pixel-by-pixel basis) between tag-on and tag-off image data sets in 2D/3D Fourier Transform reconstructed spatial domain (i.e., the result of well-known 2DFT/3DFT reconstruction processes) to provide BBTI blood perfusion images. In this case, blood perfusion (MR signal strength) as a function of time can be plotted or tabulated. For any given region of interest (e.g., an AHA segment, arbitrary operator-defined ROI or even a single pixel), a plot of the time sequence of data values provides a blood perfusion curve as a function of time. This provides several dimensions of differentiation between perfusion curves for "normal" myocardium, abnormal myocardium, ischemic myocardium, infarct myocardium - and even revascularized (treated) ischemic myocardium. As will be expected, infarct myocardium shows no perfusion (i.e., no peak or increase in detected MR signal strength). However, ischemic myocardium has a time-delayed peak flow time of occurrence, as well as weaker detected MR signal strength, when compared to normal (or revascularized) myocardium. Accordingly, ischemic areas can be distinguished based upon time and/or amplitude (or even integrated area under the curve) comparisons and/or comparisons to predetermined thresholds, etc. According to the invention, the differentiation is based on the width of the perfusion curve or the area under the perfusion curve as defined in claims 1 and 5, respectively.

A perfusion curve and/or a corresponding data table pertaining to a region of interest (e.g., a predetermined AHA myocardium segment or an arbitrary operator-defined ROI or even a single pixel) includes distinguishing characteristics so that the MRI results (e.g., as displayed to an operator or stored data for later display to others) can differently depict the ROI as representing normal, ischemic, infarct or even revascularized myocardium - all without use of any injected contrast agent (e.g., gadolinium).

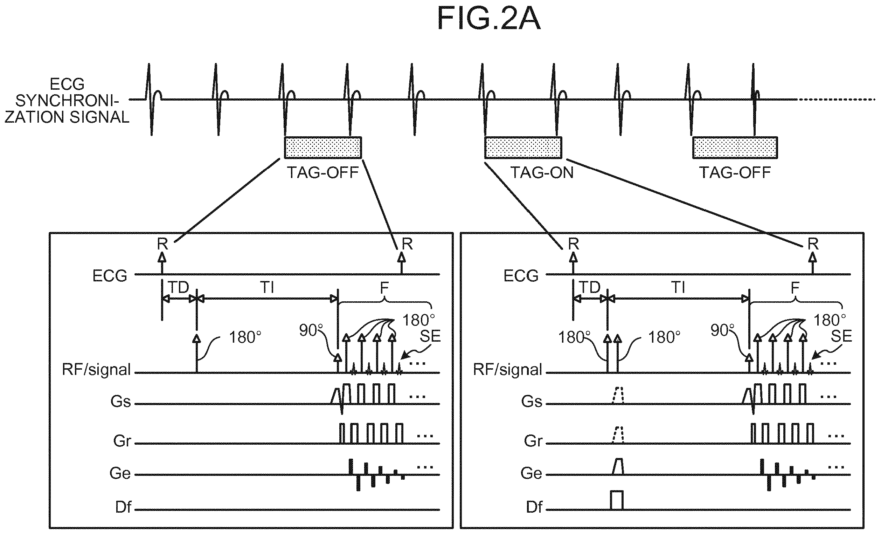

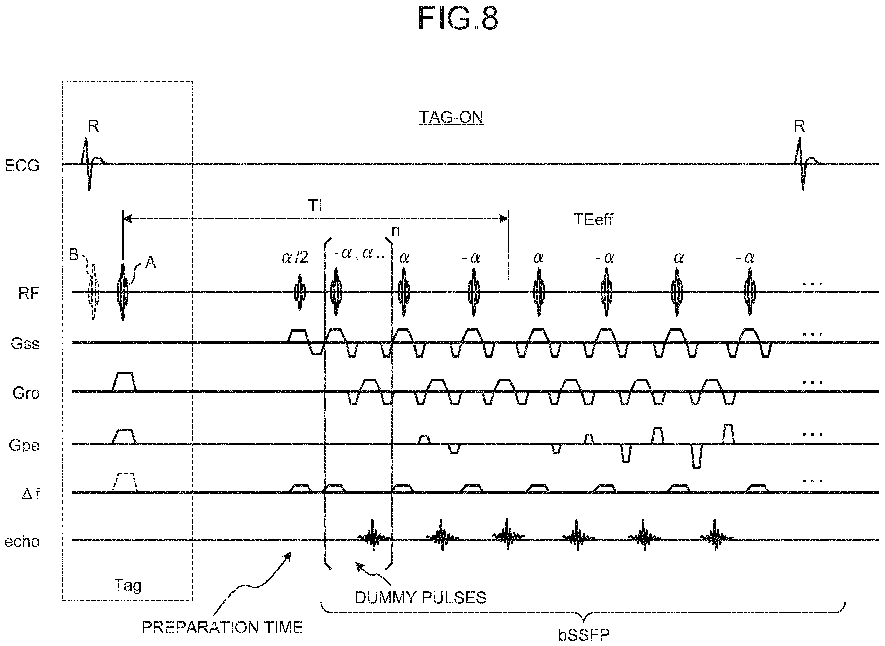

FIG. 2A is a schematic depiction of a first example of a tag-on, tag-off MRI data acquisition sequence for use in the MRI system. In a representative data acquisition sequence depicted at FIG. 2A, ECG synchronization is used so as to synchronize both "tag-off" and "tag-on" subsequences as depicted in FIG. 2A. In the tag-off subsequence shown to the left-lower side of FIG. 2A, a short initial fixed time delay TD is employed to ensure that the data acquisition subsequence begins during a desired diastole portion of the RST ECG signal. After time delay TD, a spatially non-selective 180° RF pulse is used (to help suppress background signals from a subsequent different image because the signal from myocardium is relatively small, e.g., about 10% of the total MRI signal). The actual data acquisition subsequence may, if desired, begin with a preparation pulse (e.g., a fat suppression pulse), followed by a desired data acquisition subsequence F initiated with a slice-selective (e.g., α° - typically 45° or 90°) NMR pulse to initiate a desired MRI data acquisition subsequence F, e.g., a sequence of slice-selective 180° RF refocusing pulses to elicit intervening RF spin echo (SE) responses during readout gradient Gr pulses. Each spin echo is preceded by a phase encoding Ge gradient pulse (which is varied for different echoes so as to elicit respectively corresponding lines in k-space). As those in the art will appreciate, such known MRI data acquisition subsequences might be, e.g., of the bSSFP (balanced steady-state free precession) type (which is presently preferred as shown in FIGS. 8-10) or of the fast spin echo (FSE) type or possibly of other types.

The tag-on data acquisition subsequence depicted at the right-lower side of FIG. 2A is similar to the above-described tag-off subsequence - except that, after a delay time TD, there is also a spatially selective "tagging" 180° RF pulse (e.g., perhaps the pulse at an oblique angle as represented by the dotted concurrent gradient pulses and the Df frequency offset pulse as depicted in FIG. 2A). As those in the art will appreciate, this will, in effect, revert a predetermined inflowing volume of blood back to a non-inverted magnetization orientation - thus "tagging" this inflowing volume of blood so that it will generate different MR signal responses than for the tag-off subsequence as the RF tagged flowing blood MR nuclei enter into the downstream region of interest (ROI).

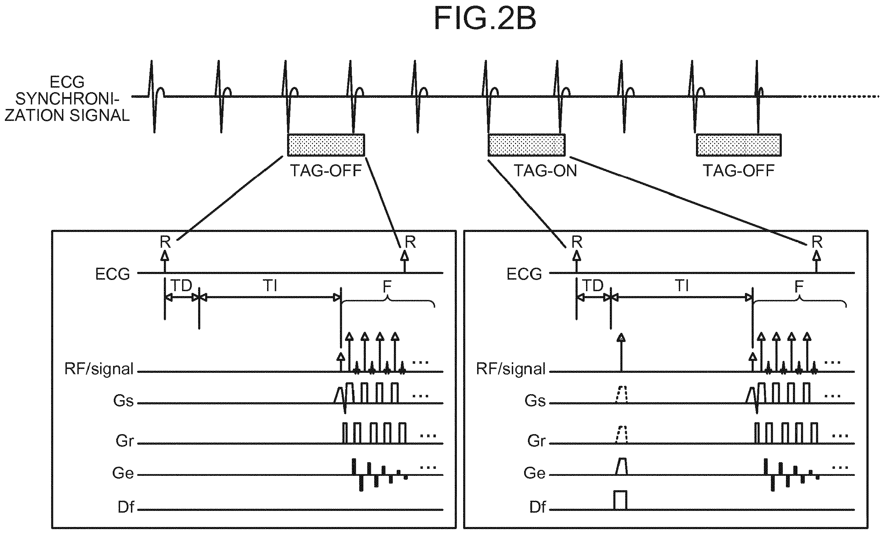

FIG. 2B is a schematic depiction of a second example of a tag-on, tag-off MRI data acquisition sequence for use in the MRI system. The tag-on/tag-off alternate acquisition subsequences depicted in FIG. 2B are the same as those depicted in FIG. 2A, except that, as will be observed, there is no initial spatially non-selective 180° pulse (in either tag-on or tag-off subsequences) for background suppression purposes.

As depicted in dotted lines in both FIGS. 2A and 2B, to achieve a desired oblique orientation for the spatially selective 180° tag-on pulse, there may be concurrent usage of differently chosen magnitude of Gs, Gr and Ge gradient pulses.

The MRI data acquisition according to the exemplary embodiment described above will be described once again. In the exemplary embodiment, the MRI system acquires sets of images (sets of blood perfusion images) depicting dynamics of blood supplied to a myocardial tissue by using a method of labeling (tagging) blood, i.e., a non-contrast method. The MRI system analyzes the acquired sets of images to obtain information for differentiating between normal, abnormal, ischemic, infarct and revascularized myocardial tissues.

The method of labeling blood will be briefly described. For example, to acquire an image depicting blood supplied to a myocardial tissue, the MRI system applies an inversion pulse that causes 180° inversion of the longitudinal magnetization as a tag pulse for labeling the blood to a region in an upper stream of the myocardial tissue where blood flows. The blood applied with the inversion pulse has a signal strength that enables differentiation from other blood and background tissues in the longitudinal magnetization recovery process. The length of time between the inversion pulse application timing and MR signal acquisition timing is typically proportional to the distance that blood applied with the inversion pulse travels. Thus, by acquiring MR signals while changing the length of time between the inversion pulse application timing and the MR signal acquisition timing, sets of images depicting dynamics of blood flowing in and flowing out of the myocardial tissue can be obtained. There is also a method where a spatially non-selective pulse without spatial selection is applied, almost simultaneously with the application of an inversion pulse, to cause 180° inversion of the longitudinal magnetization over the imaging region. In this case, the upstream blood in the myocardial tissue is applied with the inversion pulse for two times, and thus has a signal strength that enables differentiation from other blood and background tissues. The method where a spatially non-selective pulse is applied is shown in FIG. 2A and the method where no spatially non-selective pulse is applied is shown in FIG. 2B. The tag pulse is not limited to the inversion pulse that causes 180° inversion of the longitudinal magnetization.

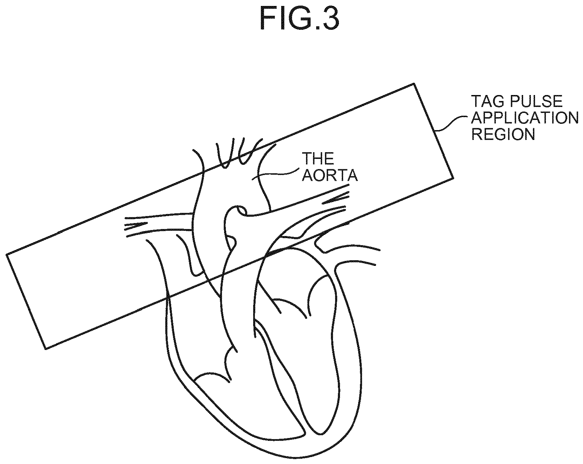

FIG. 3 is a diagram of a region to which a tag pulse is applied in the exemplary embodiment. Blood is flown out of the left ventricle into the whole body via the aorta shown in FIG. 3. Although not illustrated in FIG. 3, the left and right arteries to which the aorta bifurcates are the left coronary artery and the right coronary artery, and the left coronary artery further bifurcates into a blood vessel descending to the front side and a blood vessel coming around to the back. Blood is supplied to myocardial tissues via the three blood vessels: the right coronary artery (RCA), the left anterior descending coronary artery (LAD), and the left circumflex coronary artery (LCX). Because the exemplary embodiments are aimed at observing and analyzing dynamics of blood flowing in and out of a myocardial tissue, the region to which a tag pulse is applied is preferably set around the aorta above the heart as shown in FIG. 3.

In the exemplary embodiment, not only tag-on image data of labeled blood but also tag-off image data of unlabeled blood are acquired and a subtraction image between the tag-on and tag-off image data will be analyzed, because subtraction between the tag-on image data and tag-off image data results in subtraction of signals other than that of blood in which the tag pulse is applied and accordingly the blood in which the tag pulse is applied can be analyzed more accurately.

In the exemplary embodiment, the method is for acquiring both tag-on image data and tag-off image data in a series of pulse sequences without standby time (without operational interposition by the operator) and thus the sequences are referred to as "tag-on data acquisition subsequence", "tag-off data acquisition subsequence" etc. The difference between FIGS. 2A and 2B is in whether to once invert (FIG. 2A) or not invert (FIG. 2B) the longitudinal magnetization over the whole imaging region with a spatially non-selective pulse. In the former case, the background signal is a low signal. In view of subsequent generation of a difference image between a tag-on image and a tag-off image, the former method where the effect of misregistration between tag-on and tag-off images is small is desirable.

As will be observed in comparison between the left or the right views in FIG. 2A and the left and the right views in FIG. 2B, the difference between "tag-on data acquisition subsequence" and "tag-off data acquisition subsequence" is in whether or not to apply a spatially selective inversion pulse. As shown in FIG. 2A, if a spatially non-selective pulse is also applied, a spatially non-selective pulse and a spatially selective pulse are typically applied almost simultaneously. Thus, "TI" represents, in FIGS. 2A and 2B, the time since the spatially non-selective pulse application timing until the start of the data acquisition timing, which may be considered as, approximately, the time since the spatially selective pulse application timing until the start of the data acquisition timing

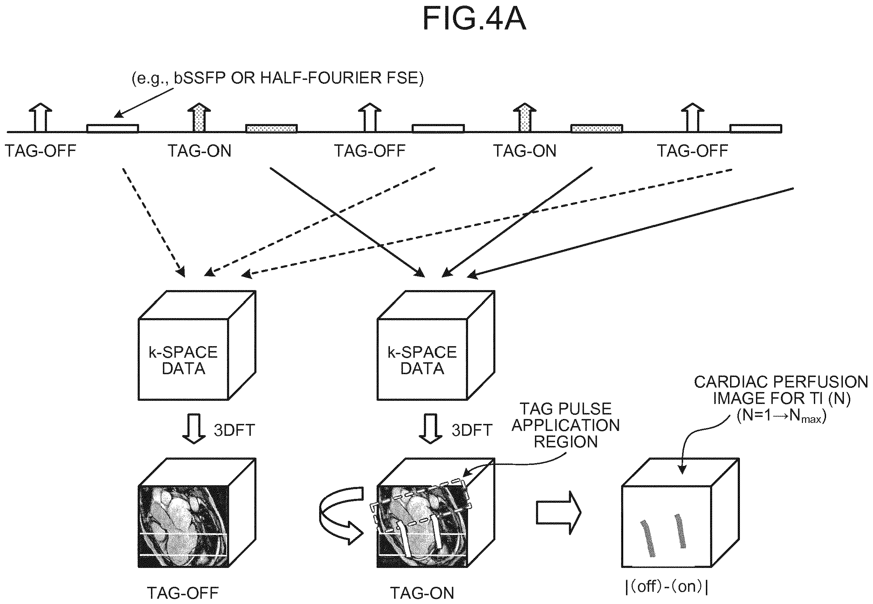

In the exemplary three-dimensional data acquisition sequence of alternate tag-on/tag-off subsequences depicted at FIG. 4A, as those in the art will appreciate, sufficient k-space data is eventually gathered for both tag-off and tag-on conditions to permit tag-on and tag-off image reconstructions into the spatial domain (e.g., using a three-dimensional Fourier Transform technique well known in the art if a 3D data acquisition sequence has been utilized). The difference between the reconstructed tag-off and tag-on images for a desired region of interest then results from differencing the spatial domain tag-on/tag-off images (in either order of subtraction , the absolute value of the difference will be utilized). This results in a difference image representing cardiac perfusion for some particular TI value. As depicted in FIG. 4A, each cardiac perfusion image is for a particular TI(N) value. However, the same process will be repeated for multiple different TI values (i.e., as N varies from one to the maximum number). As will be appreciated, the TI values utilized and the intervals between them can be among the operator-controlled parameters at the outset of the entire image process.

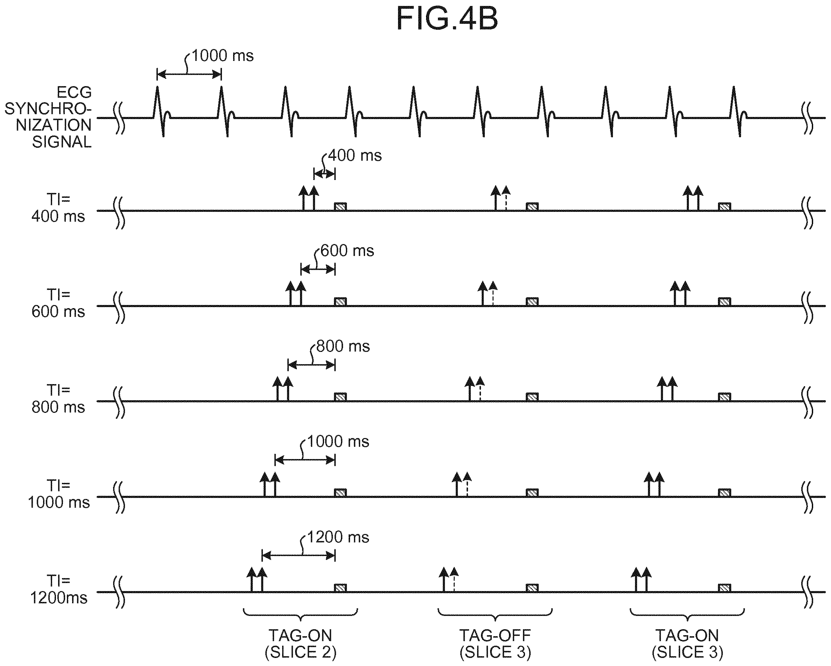

FIG. 4B is a schematic depiction of a tag-on and tag-off MRI data acquisition sequence that is executed for multiple TI times. As shown in FIG. 4B, in the exemplary embodiment, the MRI sequence controller 30 executes alternate tag-on/tag-off sub sequence for different lengths of multiple TI times. For example, as shown in FIG. 4B, the MRI sequence controller 30 executes alternate tag-on/tag off subsequences for the respective TI times: TI=400 ms, TI=600 ms, TI=800 ms, TI=1,000 ms, and TI=1,200 ms. The subsequences for different TI times may be executed as a series of pulse sequences without standby time (without operational interposition by the operator) or may be executed with appropriate insertion of standby time. The order of TI times can be arbitrarily changed.

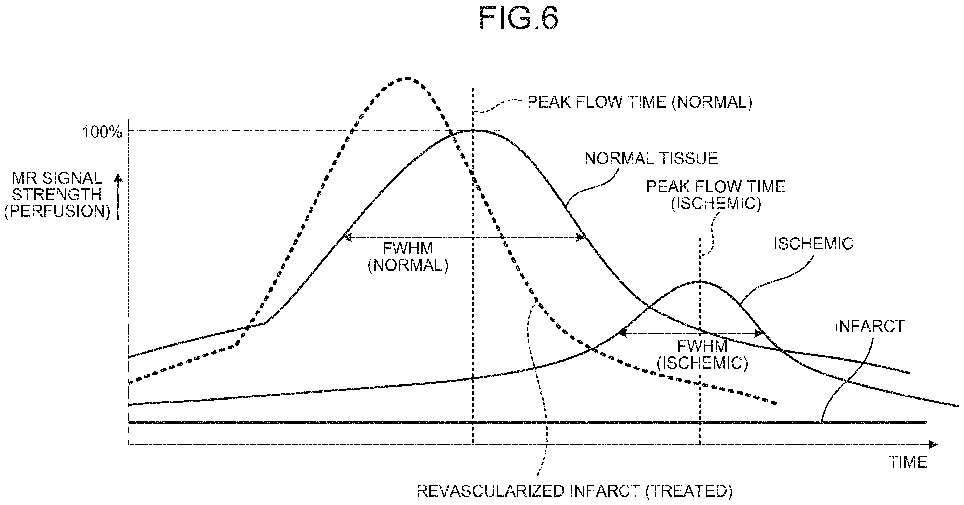

How long TI time or how long interval between TI times is used for the acquisition can be properly changed as well. For example, as shown in FIG. 6 described below, if the peak of signal strength is used as information for differentiating the state of myocardial tissues, for example, the interval may be adjusted so as to, for example, acquire data with relatively short intervals (e.g., 50-ms intervals) during a period when the signal strength is likely to be peaked, and acquire data with relatively long intervals (e.g., 200-ms intervals) during other periods, etc.

These TI times may be set by accepting inputs from an operator on a GUI for accepting imaging conditions, or set in one of pre-sequences. Normally, an examination using an MRI system includes a set of imaging sequences for acquiring images for various types of diagnosis and a set of pre-sequences executed prior to the set of imaging sequences, and a series of these sets of sequences are sequentially executed successively operator's operations, etc. being interposed For example, by making an operator to choose MR images, displaying them parallelly on the GUI by acquiring respective MR signals for each different TI times with the TI times being changed, or by making an operator to ask for the profile of the line ROI(Region of Interest) on the MR images, the pre-sequences for setting Tl times are the pre-sequences for setting appropriate TI to be used for image sequences later. Such a pre-sequence can be referred to as BBTI-prep etc. The sets of pre-sequences include, in addition, a sequence for acquiring a positioning image, a sequence for adjusting non-uniformity in the magnetic field, a sequence for acquiring a coil sensitivity map, etc.

Here, as shown in FIG. 4B, in the exemplary embodiment, there is an attempt to have the same cardiac phase of the data to be acquired between subsequences for different TI times. For example, the MRI sequence controller 30 acquires data for any TI time in a diastolic cardiac phase. In this case, spatially non-selective pulse and spatial-selective pulse application timing can be obtained by calculating back by TI times from the data acquisition timing.

For example, the diastole timing can be specified from the pre-indicated cardiac cycle of a patient. The timings obtained by tracing back on the time axis by 400 ms, 600 ms, 800 ms, 1,000 ms, 1,200 ms from the diastole timing serve as an application timing of a spatially non-selective pulse or spatially selective pulse. The spatially non-selective pulse and spatially selective pulse are applied using an R wave of an ECG synchronization signal as a trigger signal. It is sufficient if an R wave positioned prior to the obtained application timing on the time axis is specified and the elapsed time from the R wave may be set as a delay time TD. FIG. 4B shows an example where one repetition (TR (Repetition Time)) is 3R-R, but embodiments are not limited to this.

In the exemplary embodiment, the method of acquiring sets of tag-on image data and sets of tag-off image data respectively is described. In the exemplary embodiment, the method of alternately acquiring a set of tag-on image data and a set of tag-off image data for each slice or each slice encode is described, but embodiments are not limited to this. For example, a set of tag-off image data may be acquired after a set of all tag-on image data is acquired, or vice versa. In the exemplary embodiment, the method is described for acquiring tag-off image data sets for all TI times, but embodiments are not limited to this. An acquired set of tag-off image data for a TI time may be used to calculate a difference image for another TI time.

In the exemplary embodiment, the method of successively acquiring sets of image data for different TI times in a series of pulse sequences without standby time (without operational interposition by the operator), but embodiments are not limited to this. For example, a standby time may be inserted every time when the TI time is changed. In the exemplary embodiment, the method of acquiring image data in the diastolic cardiac phase in synchronization with an ECG synchronization signal, but embodiments are not limited to this and image data may be acquired using another cardiac phase. Furthermore, the acquiring method of the exemplary embodiment shown in FIGS. 2A to 4A is not limited to them and may be changed arbitrarily.

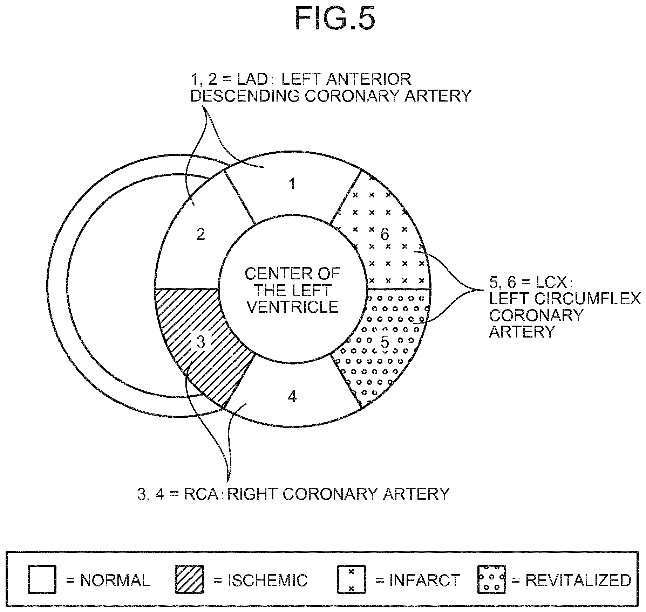

FIG. 5 is a schematic depiction of myocardial tissue segmentation of a left ventricular short axis cross-sectional image, depicting an AHA model. In the exemplary embodiment, for example, the MRI system controller 22 stylizes the result of analyzing image data and displays it on the display 24. An example thereof will be described using FIG. 5. The numbered segments 1 to 6 in FIG. 5. are previously defined so as to represent specified portions of myocardium. This type of segmented display is commonly referred to as a "Bull's Eye" display - and multiple such can be superimposed in concentric fashion (e.g., to concurrently depict base, middle and upper short-axis cross-sections of myocardium). If these segments have been chosen as regions of interest (e.g., as may be chosen so in the default setting of a MRI system ), then the exemplary embodiments permit these different regions of interest to be depicted differently on the screen of the display 24 viewed by an operator/doctor and/or as represented by various ways, such as by a table . For example, as shown in FIG. 5, a segment 3 has been determined and displayed as an ischemic myocardium tissue, while segment 6 has been determined and displayed as an infarct myocardium tissue, while segment 5 has been determined and displayed as a revitalized myocardium tissue- while all other displayed segments are "normal".

The example of a display of an analysis result of the exemplary embodiment will be described once again. As described below, in the exemplary embodiment, the MRI system controller 22 (also referred to as an "analyzer") analyzes image data sets acquired for multiple different TI times and analyzes changes in the signal strength as time passes (i.e., change of TI time) after the tag-pulse application, thereby acquiring information for differentiating between the states, such as normal and abnormal tissues, of the myocardial tissue to be analyzed. The analysis may be performed on, for example, an individual region of interest (ROI) in the myocardium specified by an operator or performed for each set of pixels. In other words, this analysis is performed on an arbitrary region segmented in the myocardium. FIG. 5 depicts an AHA model as an example of regions segmented in the myocardium.

As described above, blood is supplied to myocardial tissues via three blood vessels: the right coronary artery (RCA), left anterior descending coronary artery (LAD), and left circumflex coronary artery(LCX). Furthermore, as shown in FIG. 5, among the myocardial tissues, the segments 1 and 2 are supplied with blood via the left anterior descending coronary artery, the segments 3 and 4 are supplied with blood via the right coronary artery, and the segments 5 and 6 are supplied with blood via the left circumflex coronary artery.

For example, the MRI system controller 22 analyzes changes in the signal strength for each segment. In other words, for example, the MRI system controller 22 first analyzes the signal strength of each pixel as described below and then calculates a mean value of analysis results for the respective pixels of each segment, thereby calculating an analysis result for each segment. Calculation of a mean value is a mere example. According to the analysis result, the MRI system controller 22 differentiates segments between normal, ischemic, infarct and revascularized infarct myocardial tissues. The MRI system controller 22 creates a stylized analysis result by superimposing patterns corresponding to the differentiated tissues on the respective segments of the AHA model and displays it on the display 24. For example, in the example in FIG. 5, the segment 3 is depicted in an ischemic myocardial tissue pattern, the segment 5 is depicted in a revascularized infarct myocardial tissue pattern, and the segment 6 is depicted in an infarct myocardial tissue pattern. The segments 1, 2 and 5 are depicted in a normal myocardial tissue pattern.

Regarding the example in FIG. 5, the method of distinguishing the difference between tissues in different states by their patterns is described, but embodiments are not limited to this. For example, a distinguishing method using the color difference or a distinguishing method using letters (e.g., "ischemic", etc.) may be employed. Regarding the example in FIG. 5, the example of superimposing patterns on the stylized AHA model is described. However, embodiments are not limited to this. For example, an analysis result may be superimposed by using patterns, colors, or letters on a short axis cross-sectional image of a reconstructed MR image. Regarding this superimposition, display can be switched on/off if necessary according to an operation by an operator.

Subsequently, the analysis of sets of image data acquired for multiple different TI times will be described. FIG. 6 is an exemplary plot diagram where acquired MR signal strength (representing blood perfusion) is plotted as a function of time. In the exemplary embodiments, such differentiation between these types of myocardial tissue, without use of contrast agents, becomes possible because the perfusion data acquired using variable TI times in a BBTI technique provides sufficient data to, in effect, plot the received MR perfusion signal strength for any desired region of interest (ROI) as a function of time, as depicted schematically at FIG. 6. As shown in FIG. 6, a normal myocardial tissue has a certain detectable peak flow time as represented by the mid-point of its full-width at half-maximum (FWHM) dimension - while an ischemic myocardial tissue has a temporally displaced later peak flow time, a smaller amplitude and smaller FWHM. Of course, the infarct myocardial tissue, as would be expected, exhibits no peak at all (if, in fact, there is not any detectable perfusion response at all). At the same time, as depicted in FIG. 6, revascularized previously infarct myocardial tissue (i.e., after treatment such as by installation of stents and/or surgical bypass procedures) can be expected to have a yet earlier peak flow time in the temporal domain and possibly a slightly higher peak flow level. As will be appreciated from FIG. 6, the integrated area under the curve (e.g., flow quantity) may also be used to distinguish between different kinds of myocardial tissue.

According to the invention, the method of distinguishing between different types of myocardium tissues relies upon the width of the perfusion curve or the area under the perfusion curve as defined in claims 1 and 5, respectively. However, it will be appreciated that embodiments of the invention or examples that do not form part of the claimed invention may rely upon peak flow temporal distinctions, the peak-flow level and/or integrated area under the curve and/or various combinations and permutations of these three possible distinguishing characteristics to distinguish between these different types of myocardial tissue - all without using an injected chemical contrast agent.

Normal mean transit time, calculated from the FWHM, and the peak flow time can be tabulated for any specified region of interest (ROI). As those in the art will appreciate, the acquired data points can be best-fitted to a curve and/or directly analyzed (e.g., to provide FWHM/peak time measurements) using conventional curve fitting and analysis techniques. Arbitrary signal strength units can be indicated as a percent of the normal signal, which is assumed to be, e.g., 100%. As a result of such myocardium analysis, a table of peak flow time (e.g., FWHM) and area under the perfusion curve can be provided for each AHA myocardium segment, as well as any other desired and specified ROI #1, ROI #2, and so on (even down to a single pixel, if desired).

For example, if the perfusion difference image data is computed on a pixel-by-pixel basis, then the difference values for all pixels in a particular ROI can be added together or averaged or otherwise combined (or analyzed) to provide a single overall perfusion curve for that ROI. Of course, one could display all pixels of the ROI as individually shaded or colored to represent different tissue types.

The exemplary embodiments are based on non-contrast perfusion MRI where it is possible to resolve, e.g., in a few tens of milliseconds scale increments of tagged blood flow over a few hundred milliseconds (e.g., perhaps up to 1,000 ms of suitable heartbeat cycle is available). Such observation was not possible in fast pass Gd contrast perfusion MRI where the best time resolution is on the order of only a couple of seconds (e.g., 2,000 ms).

The exemplary embodiments permit depicting blood flows of a desired temporal resolution by controlling the TI time delay for different sets of data acquisition subsequences. One can use TI times of, e.g., minimum initial TI values such as 100 ms, followed by TI times of 200 ms, 300 ms, 400 ms, 500 ms, 600 ms, 800 ms, 1,000 ms and 1,200 ms. One can also use any desired different sequence of TI values, e.g., 100, 200, 250, 300, 350, 375, 400, 425, 450, 475, 500, 600, 800 and 1,000 ms. The effective range for each tag-on or tag-off subsequence is one RR interval of the ECG signal (e.g., perhaps 1,000 ms for 60 heartbeats per minute. The RR interval can be calculated using, for example, the equation: RR=60,000 ms / HR (RR=RR interval, HR=Heart Rate), which, however, do not limit equations for calculating an RR interval)). Here, for imaging using the MRI system, for example, electrocardiographic signals that are output from the electrocardiograph worn by a patient can be used as synchronous signals during imaging. The electrocardiograph further outputs an electrocardiographic waveform and heart rate (HR). For example, at the step before imaging according to the exemplary embodiment, if the heart rate can be acquired from the electrocardiograph worn by the patient (can be automatically input to the MRI system or input to the MRI system via the operator), the MRI system can substitute the acquired heart rate in the equation to calculate the RR interval. The MRI system can determine multiple TI times on a basis of the calculated RR interval. For example, the MRI system can appropriately segment the calculated RR interval by the above-described intervals to determine multiple TI times. A TI time can be set longer then the RR interval. For example, when the RR interval is 1,000 msec, the TI time can be set as 1,200 msec. Furthermore, the heart rate can be acquired not only from an electrocardiograph but also from a sphygmograph worn by a patient.

Use of the exemplary embodiments allows separation of normal myocardium from abnormal myocardium having unusual blood supply. The measurement of the peak flow (or FWHM) representing a mean transit time and/or integrated value of the area under the curve provide differentiation of normal myocardium from abnormal myocardium. Since one can achieve temporal resolution of less than 100 ms, a stress examination may not be required - and clearly Gd late contrast examination is not required. The exemplary temporal resolution allows prediction of normal myocardium from abnormal myocardium. In addition, differentiation of treated and untreated infarct myocardium can be observed.

The analysis of the exemplary embodiment described using FIG. 6 will be described once again. As described above, in the exemplary embodiment, the MRI sequence controller 30 executes data acquisition subsequences for different lengths of multiple TI times. A TI time is approximately a time from the timing of applying a tag-pulse until the timing of acquiring data in the imaging region. In other words, the longer the TI time is, the longer the time of blood perfusion to myocardial tissues is. Regardless in which order data for different TI times is acquired, the analysis results are placed on the time axis according to the order of TI times and, if, for example, curve fitting is performed, the signal strength of blood flowing in and then flowing out of the myocardial tissues can be depicted by a curve that is a function of time as shown in FIG. 6.

In the exemplary embodiment, the MRI system controller 22 analyzes the curve after the curve fitting to differentiate the state of myocardial tissues of the region of interest to which the curve corresponds. For example, the MRI system controller 22 specifies the time of the peak of the curve to obtain a "peak flow time" when the signal strength of blood flowing within the region of interest is the maximum. Furthermore, for example, the MRI system controller 22 calculates an FWHM as an index indicative of the width of curve and, according to the width of the FWHM, obtains the "transit time (peak flow amplitude)", being the time from the flowing of the blood into the region of interest until the flowing of the blood out of the region of interest. Furthermore, for example, the MRI system controller 22 calculates the area under the curve to obtain the "integrated flow quantity" that is the quantity of blood perfusing into myocardium. Here, FWHM is a mere example, and HWHM (Half Width at Half Maximum) etc. may be employed.

For example, if the tendency of "peak flow time", "peak flow amplitude" and "integrated flow quantity" has been identified, it is possible to analyze that a myocardial tissue representing a "peak flow time", "peak flow amplitude" and "integrated flow quantity" that are different from those of a normal myocardial tissue is an abnormal myocardial tissue of some sort. For example, as shown in FIG. 6, an ischemic myocardial tissue has a tendency that, compared to a normal myocardial tissue, the "peak flow time" becomes later, the "peak flow amplitude" becomes smaller, and the "integrated flow quantity" becomes smaller. Furthermore, for example, as shown in FIG. 6, a revascularized infarct tissue has a tendency that, compared to a normal myocardial tissue, the "peak flow time" becomes relatively earlier and, furthermore, the signal strength at the peak time becomes stronger. This can be considered as a result of the fact that, since instruments, such as stents, being placed on the revascularized infarct tissues, the revascularized infarct tissues tend not to be affected by heartbeats compared to normal myocardial tissues and thus blood flows easily. The analysis described here is a mere example and what kind of index is and how it is used to differentiate states of myocardial tissues can be arbitrarily changed.

The MRI system controller 22 may display a graph, like that shown in FIG. 6, depicting changes in the signal strength on the display 24 as an example of displaying analysis results. As shown in FIG. 6, the MRI system controller 22 may display curves of multiple regions of interest collectively (in a superimposed manner) in one graph. When displaying a graph, the MRI system controller 22 may also display analysis results ("normal tissue", "ischemic", "infarct" and "revascularized infarct (treated)") as shown in FIG. 6 or also display segment names (e.g., "segment 1" and "segment 2"), etc. The MRI system controller 22 may appropriately change the display mode by displaying both of them, by color coding, by line-type coding, etc. In the exemplary embodiment, the MRI system controller 22 analyzed the signal change and differentiated between myocardial tissues. However, embodiments are not limited to this. For example, differentiation between myocardial tissues is not an essential configuration. For example, the MRI system controller 22 may analyze the signal change and displays a graph and an operator, such as a doctor, may observe the graph displayed on the display 24 to perform differentiation.

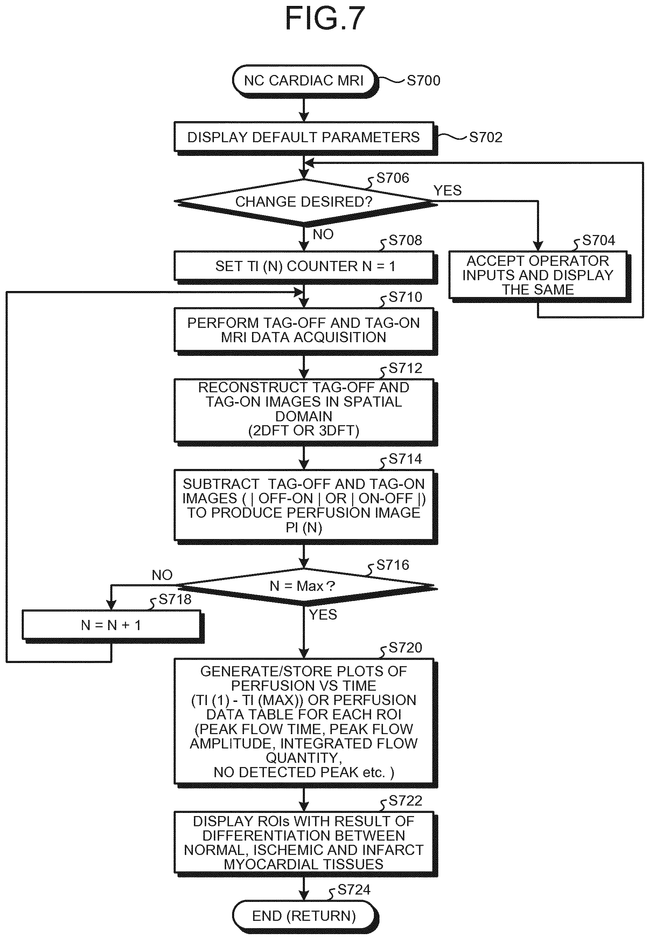

FIG. 7 is a flowchart illustrating a computer program code structure for implementing exemplary embodiments of non-contrast myocardial MRI. The "non-contrast cardiac MRI" shown in FIG. 7 is performed as one imaging sequence in a series of sets of sequences. Needless to say, for example, the image generation process performed at the following steps S712 to S714 or the analysis process, the display process, etc. performed at or at a later step than S720 is not necessarily performed in one imaging sequence. For example, they may be performed as one of post-processes after execution of all sets of imaging sequences.

In accordance with the stored program data code structures of FIG. 1, the MRI system can enter the non-contrast cardiac MRI subroutine depicted at FIG. 7 via an appropriate over-arching operating system or the like (step S700), as will be well understood by those in the art. Once the non-contrast cardiac MRI subroutine being entered, the MRI system may display any number of desired default parameters on the display 24 (step S702). For example, default parameters might specify possible data acquisition subsequences as being of the bSSFP type or the half-Fourier FSE or otherwise. Furthermore, the default parameters might include designation of whether or not a spatially non-selective 180° pulse might be employed initially (in both tag-on and tag-off subsequences) so as to suppress background signals (as depicted in FIG. 2A as contrasted with FIG. 2B) in the final perfusion image. One may also designate whether 2D acquisition is desired or 3D data acquisition is desired. Of course, regions of interest can be designated manually or, perhaps, the standard AHA heart segments could be defaulted to as regions of interest. Whether one wants to use temporal discrimination, amplitude discrimination and/or integrated area under the curve discrimination for differentiating between normal, ischemic, infarct and revascularized infarct myocardia may be designated. The number of TI times and/or the value of such times may be among the default parameters that could be adjusted by the operator, if desired, at step S704 (if such desire is selectively determined at step S706).

Once non-contrast cardiac MRI subroutine parameters have been determined (whether by default or by operator setting), then the TI counter N is initialized at step S708. The MRI system performs tag-off/tag-on MRI data acquisitions for k-space for that particular TI(N) value (step S710). The MRI system then uses such k-space MRI data to suitably achieve reconstruction into the spatial domain (step S712). Then, the MRI system subtracts the generated tag-off/tag-on images to produce a perfusion image data for the current TI(N) value (step S714).

If the TI counter N has not yet reached its maximum value (NO at step S716), then the MRI system increments the counter (step S718) and repeats the processes at step S710 to S714 for another TI value.

Eventually, N will be equal to the maximum value (YES at step S716) and the MRI system passes control to step S720 in FIG. 7. Here, acquired perfusion image data as a function of time is used to generate/store plots of perfusion vs. time data and/or perfusion data table entries for each region of interest (e.g., peak flow times, peak flow amplitudes, integrated flow quantity, lack of detected peak, etc.). Thereafter, the MRI system displays the regions of interest with results of differentiation between normal, ischemic and infarct myocardium values (e.g., see FIG. 5). If desired, the MRI system can superimpose perfusion curves for different regions of interest. The MRI system can at step S724 leave the non-contrast cardiac MRI back to the higher level calling program code structure.

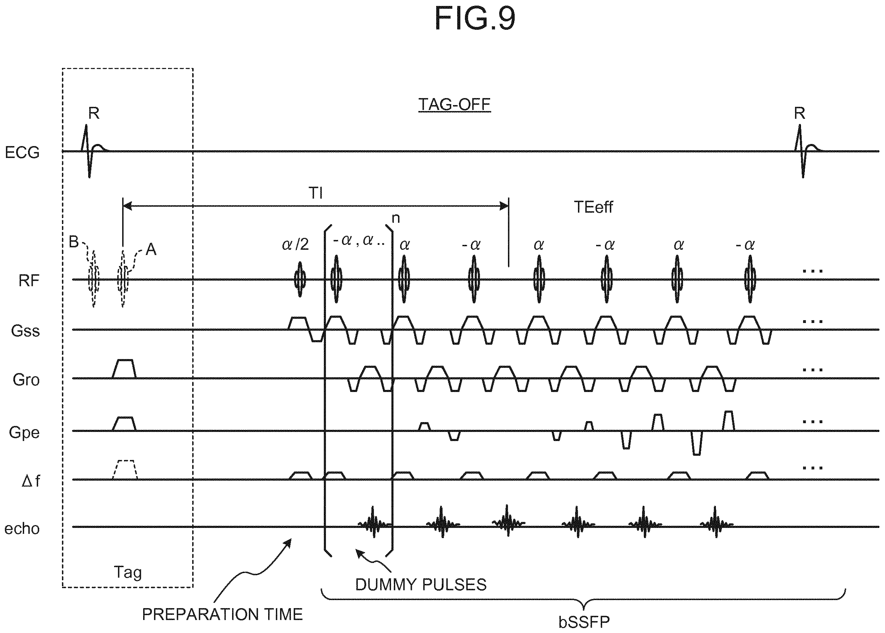

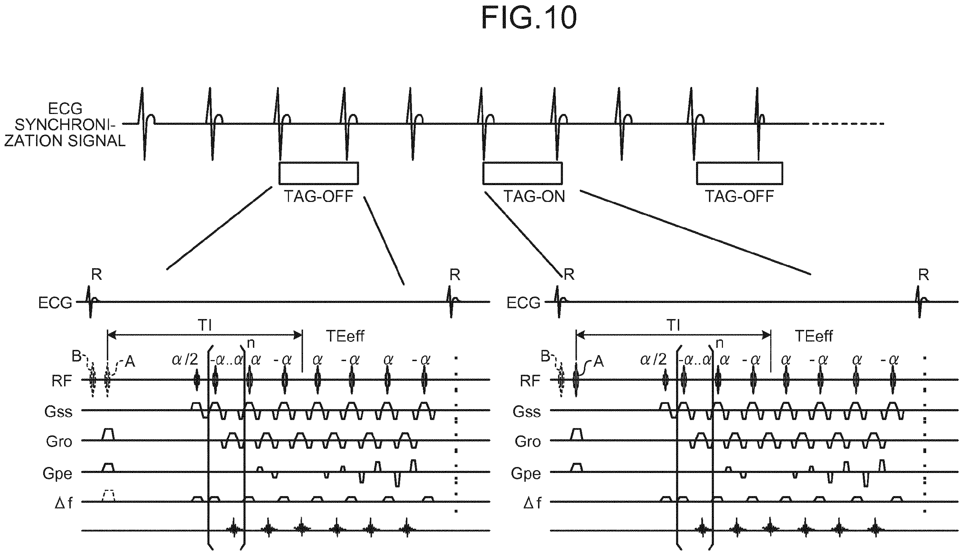

Although single shot FSE or FASE (Fast Asymmetric Spin Echo or Fast Advanced Spin Echo) data acquisition sequences can be used as the actual data acquisition subsequence, the presently preferred data acquisition subsequences are bSSFP sequences. FIG. 8 is a schematic depiction of a bSSFP tag-on MRI data acquisition sequence, FIG. 9 is a schematic depiction of a bSSFP tag-off MRI data acquisition sequence, and FIG. 10 is a schematic depiction of a tag-on/tag-off alternating MRI data acquisition sequence.

For example, in FIG. 8 a tag-on subsequence is depicted for perfusion flow-in using a spatially selective RF tag pulse with 2D/3D bSSFP. The spatially selective RF tag pulse A is depicted in solid line while the optional spatially non-selective inversion pulse B (for background suppression as previously noted) of which application is arbitrarily selected is depicted in dotted line. FIG. 9 depicts a similar tag-off subsequence (where the position of the tag pulse for the tag-on sequence is also shown in dotted line for reference purposes). Notice that the same gradient magnetic pulses may be used at position A so as to maintain uniformity in this respect between the tag-on and tag-off subsequences (even though there is no spatially selective RF tag pulse A actually employed for the tag-off subsequence). FIG. 10 depicts the preferred tag-on/tag-off alternate acquisition process (i.e., in effect repeatedly concatenating a tag-Off subsequence (e.g., FIG. 9) and a tag-on subsequence (e.g., FIG. 8).

In the above-described exemplary embodiments, the MRI system acquires MR signals in both of (a) tag-on data acquisition subsequences using spatially selective RF pulses (tag pulses) and (b) tag-off data acquisition subsequences not using spatially selective RF pulses (tag pulses). The MRI system combines them to obtain MR image data. Here, the MRI system acquires MR signals for multiple TI times in multidimensional tag-on and tag-off data acquisition subsequences without use of any injected contrast agent. The MRI system subtracts the acquired sets of MR image data for each TI time and acquires difference values that are difference values as a function of time indicative of blood perfusion within a region of interest and that differentiate between normal, ischemic and infarct myocardial tissues.

In the exemplary embodiments, the example is described where the MRI system performs data acquisition using tag-on and tag-off MRI data acquisition sequences and analysis using the acquired data, but embodiments are not limited to this. For example, image processing apparatuses other than MRI systems can perform the analysis process. In other words, an image processing apparatus may have the same functions as those of the analyzer and use the data acquired by an MRI system to analyze changes in the signal strength over time since application of tag pulse. The image processing apparatus includes various types of apparatuses, such as, a work station, an image storage apparatus (image server) and a viewer of a PACS (Picture Archiving and Communication System), and an electric health record system.

The magnetic resonance imaging apparatus of the embodiments can acquire information that enables differentiation between normal, ischemic, infarct, and revascularized infarct tissues in the myocardium of a patient.

While certain embodiments have been described, these embodiments have been presented by way of example only, and are not intended to limit the scope of the inventions. Indeed, the novel embodiments described herein may be embodied in a variety of other forms; furthermore, various omissions, substitutions and changes in the form of the embodiments described herein may be made without departing from the invention as long as such forms or modifications fall within the scope of the appended claims.

## Claims

1. A magnetic resonance imaging apparatus comprising:
a sequence controller (30) configured to perform multiple data acquisitions in an imaging region, each of the multiple data acquisitions being performed after each of multiple given times since each of the applications of a tag pulse to a tag region, with the multiple given times being different to each other, wherein the imaging region includes the myocardium of a patient and the tag region is set in an upper blood stream of the myocardium; and
an analyzer (22) configured to use multiple sets of data corresponding to the multiple given times to analyze changes in signal strength of blood in the myocardium over time since each of the applications of the tag pulse; wherein the analyzer (22) is configured to analyze the changes in the signal strength, for each of multiple segments comprising a plurality of pixels by combining the plurality of pixels and thus configured to output information that enables differentiation between normal, ischemic, infarct, and revascularized infarct tissues for each of the multiple segments to be analyzed, wherein
the sequence controller (30) is further configured to perform data acquisition to acquire data of an imaging region without applying the tag pulse,
the analyzer (22) is configured to calculate a difference image between a first image that is reconstructed from data for the imaging region, with the data being acquired with application of the tag pulse for one of the multiple given times, and a second image that is reconstructed from data for the imaging region, with the data being acquired without application of the tag pulse,
configured to calculate, from a curve representing changes in the signal strength of calculated difference images over time since the application of the tag pulse, an index indicative of a width of the curve and configured to output the information according to the calculated index.

2. The magnetic resonance imaging apparatus according to claim 1, wherein the analyzer (22) is configured to display on a display (24) a graph representing the changes in the signal strength.

3. The magnetic resonance imaging apparatus according to claim 1, wherein the analyzer (22) is configured to derive, according to the index, at least one of a time at which blood tagged by the tag pulse passes through the imaging region and a time at which the signal strength of blood tagged by the tag pulse flowing through the imaging region is at a peak.

4. The magnetic resonance imaging apparatus according to claim 1, wherein the analyzer (22) is configured to generate an analysis result indicating a result of the differentiation, with the result of the differentiation being superimposed on an image of the region in the site to be analyzed and wherein the analyzer (22) is configured to display the generated analysis result on a display (24).

5. A magnetic resonance imaging apparatus comprising:
a sequence controller (30) configured to perform multiple data acquisitions in an imaging region, each of the multiple data acquisitions being performed after each of multiple given times since each of the applications of a tag pulse to a tag region, with the multiple given times being different to each other, wherein the imaging region includes the myocardium of a patient and the tag region is set in an upper blood stream of the myocardium; and
an analyzer (22) configured to use multiple sets of data corresponding to the multiple given times to analyze changes in signal strength of blood in the myocardium over time since each of the applications of the tag pulse; wherein the analyzer (22) is configured to analyze the changes in the signal strength, for each of multiple segments comprising a plurality of pixels by combining the plurality of pixels and thus configured to output information that enables differentiation between normal, ischemic, infarct, and revascularized infarct tissues for each of the multiple segments to be analyzed, wherein
the sequence controller (30) is further configured to perform data acquisition to acquire data of an imaging region without applying the tag pulse,
the analyzer (22) is configured to calculate a difference image between a first image that is reconstructed from data for the imaging region, with the data being acquired with application of the tag pulse for one of the multiple given times, and a second image that is reconstructed from data for the imaging region, with the data being acquired without application of the tag pulse,
configured to calculate, from a curve representing changes in the signal strength of calculated difference images over time since the application of the tag pulse, an area under the curve and configured to output the information according to the calculated area under the curve.

## Patentansprüche

1. Magnetresonanz-Bildgebungsgerät, Folgendes umfassend:
eine Sequenzsteuerung (30), welche konfiguriert ist, um multiple Datenerfassungen in einem Bildgebungsbereich durchzuführen, wobei jede der multiplen Datenerfassungen nach jeder von multiplen gegebenen Zeiten ab jeder der Anwendungen eines Markierungsimpulses auf einen Markierungsbereich durchgeführt wird, wobei die multiplen gegebenen Zeiten sich jeweils voneinander unterscheiden,
wobei der Bildgebungsbereich das Myokard eines Patienten einschließt und der Markierungsbereich in einem oberen Blutstrom des Myokards festgelegt ist; und
einen Analysator (22), welcher konfiguriert ist, um multiple Datensätze zu verwenden, welche den multiplen gegebenen Zeiten entsprechen, zum Analysieren von Änderungen der Signalstärke von Blut in dem Myokard im Zeitverlauf ab jeder der Anwendungen des Markierungsimpulses;
wobei der Analysator (22) konfiguriert ist, um die Änderungen der Signalstärke für jedes von multiplen Segmente zu analysieren, welche eine Vielzahl von Pixeln umfassen, durch Kombinieren der Vielzahl von Pixeln, und folglich konfiguriert ist, um Informationen auszugeben, welche eine Differenzierung zwischen normalen, ischämischen, Infarkt-und neuvaskularisierten Infarktgeweben für jedes der multiplen zu analysierenden Segmente ermöglicht, wobei
die Sequenzsteuerung (30) ferner konfiguriert ist, um eine Datenerfassung durchzuführen, um Daten eines Bildgebungsbereichs ohne Anwendung des Markierungsimpulses zu erfassen,
der Analysator (22) konfiguriert ist, um ein Differenzbild zwischen einem ersten Bild zu berechnen, welches anhand von Daten des Bildgebungsbereichs rekonstruiert wurde, mit den mit Anwendung des Markierungsimpulses für eine der multiplen gegebenen Zeiten erfassten Daten, und einem zweiten Bild, welches anhand von Daten für den Bildgebungsbereich rekonstruiert wurde, mit Daten, welche ohne Anwendung des Markierungsimpulses erfasst wurden,
konfiguriert ist, um anhand einer Kurve, welche Änderungen der Signalstärke berechnete Differenzbilder im Zeitverlauf ab der Anwendung des Markierungsimpulses darstellt, einen Index zu berechnen, welcher eine Breite der Kurve angibt, und konfiguriert ist, um die Informationen gemäß dem berechneten Index auszugeben.

2. Magnetresonanz-Bildgebungsgerät nach Anspruch 1, wobei der Analysator (22) konfiguriert ist, um an einem Display (24) eine Grafik anzuzeigen, welche die Änderungen der Signalstärke darstellt.

3. Magnetresonanz-Bildgebungsgerät nach Anspruch 1, wobei der Analysator (22) konfiguriert ist, um gemäß dem Index mindestens eines von einer Zeit abzuleiten, zu welcher Blut, welches durch den Markierungsimpuls markiert wurde, durch den Bildgebungsbereich passiert, und einer Zeit, zu welcher die Signalstärke des durch den Markierungsimpuls markierten Blutes, welches durch den Bildgebungsbereich strömt, an einem Peak steht

4. Magnetresonanz-Bildgebungsgerät nach Anspruch 1, wobei der Analysator (22) konfiguriert ist, um ein Analyseergebnis zu erzeugen, welches ein Ergebnis der Differenzierung angibt, wobei das Ergebnis der Differenzierung einem Bild des Bereichs an der zu analysierenden Stelle überlagert wird, und wobei der Analysator (22) konfiguriert ist, um das erzeugte Analyseergebnis an einem Display (24) anzuzeigen.

5. Magnetresonanz-Bildgebungsgerät, Folgendes umfassend:
eine Sequenzsteuerung (30), welche konfiguriert ist, um multiple Datenerfassungen in einem Bildgebungsbereich durchzuführen, wobei jede der multiplen Datenerfassungen nach jeder von multiplen gegebenen Zeiten ab jeder der Anwendungen eines Markierungsimpulses auf einen Markierungsbereich durchgeführt wird, wobei die multiplen gegebenen Zeiten sich jeweils voneinander unterscheiden, wobei der Bildgebungsbereich den Myokard eines Patienten einschließt und der Markierungsbereich in einem oberen Blutstrom des Myokards festgelegt ist; und
einen Analysator (22), welcher konfiguriert ist, um multiple Datensätze zu verwenden, welche den multiplen gegebenen Zeiten entsprechen, zum Analysieren von Änderungen der Signalstärke von Blut in dem Myokard im Zeitverlauf ab jeder der Anwendungen des Markierungsimpulses;
wobei der Analysator (22) konfiguriert ist, um die Änderungen der Signalstärke für jedes von multiplen Segmente zu analysieren, welche eine Vielzahl von Pixeln umfassen, durch Kombinieren der Vielzahl von Pixeln, und folglich konfiguriert ist, um Informationen auszugeben, welche eine Differenzierung zwischen normalen, ischämischen, Infarkt- und neuvaskularisierten Infarktgeweben für jedes der multiplen zu analysierenden Segmente ermöglicht, wobei
die Sequenzsteuerung (30) ferner konfiguriert ist, um eine Datenerfassung durchzuführen, um Daten eines Bildgebungsbereichs ohne Anwendung des Markierungsimpulses zu erfassen,
der Analysator (22) konfiguriert ist, um ein Differenzbild zwischen einem ersten Bild zu berechnen, welches anhand von Daten des Bildgebungsbereichs rekonstruiert wurde, mit den mit Anwendung des Markierungsimpulses für eine der multiplen gegebenen Zeiten erfassten Daten, und einem zweiten Bild, welches anhand von Daten für den Bildgebungsbereich rekonstruiert wurde, mit Daten, welche ohne Anwendung des Markierungsimpulses erfasst wurden, konfiguriert ist, um anhand einer Kurve, welche Änderungen der Signalstärke berechneter Differenzbilder im Zeitverlauf ab der Anwendung des Markierungsimpulses darstellt, eine Fläche unterhalb der Kurve zu berechnen,
und konfiguriert ist, um die Informationen gemäß der berechneten Fläche unterhalb der Kurve auszugeben.

## Revendications

1. Appareil d'imagerie par résonance magnétique comprenant :
un contrôleur de séquence (30) qui est configuré pour réaliser de multiples acquisitions de données dans une région d'imagerie, chacune des multiples acquisitions de données étant réalisée après chacun de multiples temps donnés depuis chacune des applications d'une impulsion d'étiquette sur une région d'étiquette, les multiples temps donnés étant différents les uns des autres,
dans lequel la région d'imagerie inclut le myocarde d'un patient et la région d'étiquette est définie dans un flux sanguin supérieur du myocarde ; et
un analyseur (22) qui est configuré pour utiliser de multiples jeux de données qui correspondent aux multiples temps donnés pour analyser des variations de l'intensité de signal du sang dans le myocarde en fonction du temps depuis chacune des applications de l'impulsion d'étiquette ;
dans lequel l'analyseur (22) est configuré pour analyser les variations de l'intensité de signal, pour chacun de multiples segments comprenant une pluralité de pixels en combinant la pluralité de pixels et est par voie de conséquence configuré pour émettre en sortie des informations qui permettent une différenciation entre des tissus normaux, ischémiques, infarcis et infarcis revascularisés pour chacun des multiples segments à analyser ; dans lequel :
le contrôleur de séquence (30) est en outre configuré pour réaliser l'acquisition de données pour acquérir des données d'une région d'imagerie sans appliquer l'impulsion d'étiquette ;
l'analyseur (22) est configuré pour calculer une image de différence entre une première image qui est reconstruite à partir de données pour la région d'imagerie, les données étant acquises au moyen de l'application de l'impulsion d'étiquette pendant l'un des multiples temps donnés, et une seconde image qui est reconstruite à partir de données pour la région d'imagerie, les données étant acquises sans l'application de l'impulsion d'étiquette,
est configuré pour calculer, à partir d'une courbe qui représente des variations de l'intensité de signal d'images de différence calculées en fonction du temps depuis l'application de l'impulsion d'étiquette, un index qui est indicatif d'une largeur de la courbe, et est configuré pour émettre en sortie les informations en fonction de l'index calculé.

2. Appareil d'imagerie par résonance magnétique selon la revendication 1, dans lequel l'analyseur (22) est configuré pour afficher sur un affichage (24) un graphique qui représente les variations de l'intensité de signal.

3. Appareil d'imagerie par résonance magnétique selon la revendication 1, dans lequel l'analyseur (22) est configuré pour dériver, en fonction de l'index, au moins un parmi un temps auquel le sang qui est étiqueté par l'impulsion d'étiquette passe au travers de la région d'imagerie et un temps auquel l'intensité de signal du sang qui est étiqueté par l'impulsion d'étiquette et qui s'écoule au travers de la région d'imagerie est à une crête.

4. Appareil d'imagerie par résonance magnétique selon la revendication 1, dans lequel l'analyseur (22) est configuré pour générer un résultat d'analyse qui indique un résultat de la différenciation, le résultat de la différenciation étant superposé sur une image de la région dans le site à analyser et dans lequel l'analyseur (22) est configuré pour afficher le résultat d'analyse généré sur un affichage (24).

5. Appareil d'imagerie par résonance magnétique comprenant :
un contrôleur de séquence (30) qui est configuré pour réaliser de multiples acquisitions de données dans une région d'imagerie, chacune des multiples acquisitions de données étant réalisée après chacun de multiples temps donnés depuis chacune des applications d'une impulsion d'étiquette sur une région d'étiquette, les multiples temps donnés étant différents les uns des autres, dans lequel la région d'imagerie inclut le myocarde d'un patient et la région d'étiquette est définie dans un flux sanguin supérieur du myocarde ; et
un analyseur (22) qui est configuré pour utiliser de multiples jeux de données qui correspondent aux multiples temps donnés pour analyser des variations de l'intensité de signal du sang dans le myocarde en fonction du temps depuis chacune des applications de l'impulsion d'étiquette ;
dans lequel l'analyseur (22) est configuré pour analyser les variations de l'intensité de signal, pour chacun de multiples segments comprenant une pluralité de pixels en combinant la pluralité de pixels et est par voie de conséquence configuré pour émettre en sortie des informations qui permettent une différenciation entre des tissus normaux, ischémiques, infarcis et infarcis revascularisés pour chacun des multiples segments à analyser ; dans lequel :
le contrôleur de séquence (30) est en outre configuré pour réaliser l'acquisition de données pour acquérir des données d'une région d'imagerie sans appliquer l'impulsion d'étiquette ; et
l'analyseur (22) est configuré pour calculer une image de différence entre une première image qui est reconstruite à partir de données pour la région d'imagerie, les données étant acquises au moyen de l'application de l'impulsion d'étiquette pendant l'un des multiples temps donnés, et une seconde image qui est reconstruite à partir de données pour la région d'imagerie, les données étant acquises sans l'application de l'impulsion d'étiquette, est configuré pour calculer, à partir d'une courbe qui représente des variations de l'intensité de signal d'images de différence calculées en fonction du temps depuis l'application de l'impulsion d'étiquette, une aire sous la courbe
et est configuré pour émettre en sortie les informations en fonction de l'aire calculée sous la courbe.
